# EUROPEAN PATENT APPLICATION

(11) **EP 3 712 895 A1**
(43) Date of publication of application: **23.09.2020**
(21) Application number: 20160375.0
(22) Date of filing: 02.03.2020
(51) Int. Cl.: G16C 20/10, G16C 20/70

(54) **BIOLOGICAL REACTION INFORMATION PROCESSING SYSTEM AND BIOLOGICAL REACTION INFORMATION PROCESSING METHOD**

(30) Priority: 18.03.2019 JP 2019050148
(71) Applicant: Hitachi, Ltd., Tokyo 100-8280 (JP)
(72) Inventor: FUJI, Taiki, Tokyo, 100-8280 (JP); ITO, Kiyoto, Tokyo, 100-8280 (JP); NAKAZAWA, Shiori, Tokyo, 100-8280 (JP); TANABE, Maiko, Tokyo, 100-8280 (JP)
(74) Representative: Strehl Schübel-Hopf & Partner

(57) **Abstract**

To predict a new biological reaction by quantifying while retaining characteristics of an entire compound structure. To provide a structural characteristic amount encoding unit that includes a conversion model unit configured to convert a characteristic amount of notation information indicating chemical structures of a plurality of compounds into a dispersedly represented numerical vector having at least two or more real number values as an element using a conversion parameter, the conversion model unit converting the characteristic amount of the notation information indicating the chemical structures into a numerical vector, for each of a first compound and a second compound among the plurality of compounds, and a biological reaction characteristic vector generator configured to generate a biological reaction characteristic vector between the first compound and the second compound by performing a calculation using a numerical vector of the first compound and a numerical vector of the second compound.

## Description

### BACKGROUND OF THE INVENTION

### 1. Field of the Invention

The present invention relates to a biological reaction information processing system and a biological reaction information processing method for a synthetic pathway design system.

### 2. Description of the Related Art

In recent years, there is an increasing need for functional organisms, which are capable of synthesizing chemicals (for example, bioplastics), pharmaceuticals (for example, antimalarial drugs), and foods (for example, functional foods) in addition to energy (for example, biofuels), by incorporating a gene sequence capable of producing a target substance into a host, with a microorganism, a plant (cell), and the like called a smart cell as the host. Advances in a biotechnology, such as a next-generation DNA sequencer technology and a genome editing technology, have enabled a creation of a variety of smart cells.

The smart cell "designs" a metabolic pathway and a genomic sequence that can synthesize the target substance, and after "synthesizing" the designed genomic sequence, selects an organism that is optimal for substance production and introduces the designed genomic sequence to create a genome-modified organism. After "measurement" of whether the genome-modified organism produces the target substance, the genome-modified organism is created by a design cycle of "learning" a measurement result and redesigning a genome sequence. In such a smart cell process, there is an increasing need for a new bioprocess using non-natural chemicals as products in addition to natural compounds produced by living organisms.

WO 2012/081723 (Patent Literature 1) is cited as related art relating to a new biological reaction information processing and a new synthetic pathway design. Patent Literature 1 discloses that a compound structure can be quantified by counting a partial structure of a given compound, and a virtual biological reaction can be quantified by creating a pair of compounds and taking a difference between the pair of compounds.

Finding a new biological reaction is important for developing a bioprocess that produces non-natural chemicals. However, it is difficult to rationally predict a new biological reaction, which depends on human knowledge, and subjects are limited to a range that humans can consider. Therefore, a method for predicting a new biological reaction that does not depend on human knowledge is desired for developing a new bioprocess.

In order to rationally design the new bioprocess, a data processing based on a knowledge and information database that is a basis is required. There is a database in which an enzymatic reaction and genetic information are accumulated as information on a synthetic pathway design of the microorganism. The database as described above can be effectively used for a top-down synthetic pathway design by data mining using accumulated data other than a bottom-up design of a related personal method.

As a method using data, there is a method using only the enzymatic reaction and metabolite information in a metabolic database. In this method, a chemical conversion rule and the like is determined based on the database, and a reaction is predicted based on the determined rule. However, the above method depends only on information in an existing database and cannot cope with predictions of a new metabolic reaction and a new pathway design. As another method, a method in which partial structures are retained and one compound structure is converted into a characteristic vector by adding partial structures together is also effective, and can be applied to a new metabolic reaction. However, the problems are that information on a positional relationship of the partial structures during vectorization is lost, and compounds other than the given partial structures cannot be taken as a subject. Therefore, various compounds can be handed in the same manner, and it is necessary to convert the biological reaction into a characteristic vector while retaining characteristics of an entire structure.

### SUMMARY OF THE INVENTION

An object of the invention is to provide a biological reaction information processing technology for a new synthetic pathway design, which is capable of predicting a new biological reaction by quantifying while retaining characteristics of the entire compound structure.

A biological reaction information processing system according to one aspect of the invention includes: a structural characteristic amount encoding unit that includes a conversion model unit configured to convert a characteristic amount of notation information indicating chemical structures of a plurality of compounds into a dispersedly represented numerical vector having at least two or more real number values as an element using a conversion parameter, the conversion model unit converting the characteristic amount of the notation information indicating the chemical structures into a numerical vector, for each of a first compound and a second compound among the plurality of compounds; and a biological reaction characteristic vector generator configured to generate a biological reaction characteristic vector between the first compound and the second compound by performing a calculation using a numerical vector of the first compound and a numerical vector of the second compound.

According to one aspect of the invention, a new biological reaction can be predicted by quantifying while retaining characteristics of an entire compound structure. Problems, configurations, and effects other than those described above will be apparent from the following description of embodiments for carrying out the invention.

### BRIEF DESCRIPTION OF THE DRAWINGS

**FIG. 1** is a configuration diagram of a biological reaction information processing device.
**FIG. 2** is a configuration diagram of a biological reaction information processing system according to a first embodiment.
**FIG. 3** is a numerical example of a biological reaction characteristic vector according to the first embodiment.
**FIG. 4** is a diagram showing a flow of generating a biological reaction characteristic vector according to the first embodiment.
**FIG. 5** is an example of a biological reaction that can be coped by the biological reaction information processing system according to the first embodiment.
**FIG. 6** is a configuration diagram of a biological reaction information processing system according to a second embodiment.
**FIG. 7** is a diagram showing a biological reaction prediction flow according to the second embodiment.
**FIG. 8** is a diagram showing an example of visualization of an analysis evaluation display unit according to the second embodiment.
**FIG. 9** is a configuration diagram in which a learning unit is provided in the biological reaction information processing system according to the second embodiment.
**FIG. 10** is a diagram showing an example of an estimation result of an enzymatic reaction using the biological reaction information processing system according to the second embodiment.
**FIG. 11** is a diagram showing a new definition of a biological reaction in a synthetic pathway design in learning of the biological reaction information processing system according to the second embodiment.
**FIG. 12** is a configuration diagram for pre-learning a structural characteristic amount encoding unit in a biological reaction information processing system according to a third embodiment.
**FIG. 13** is a configuration diagram for additionally learning the structural characteristic amount encoding unit in the biological reaction information processing system according to the third embodiment.

### DESCRIPTION OF THE PREFERRED EMBODIMENTS

Embodiments of the invention will be described below with reference to the accompanying drawings. The following description and drawings are examples for describing the invention, and are omitted and simplified as appropriate for clarification of the description. The invention can be implemented in other various forms. Unless otherwise limited, each component may be singular or plural.

In order to facilitate understanding of the invention, a position, size, shape, range, and the like of each component illustrated in the drawings may not necessarily represent an actual position, size, shape, range, and the like. Therefore, the invention is not necessarily limited to the position, size, shape, range, and the like disclosed in the drawings.

In the following description, various types of information may be described using expressions such as "table" and "list", whereas various types of information may be expressed using other data structures. "XX table", "XX list", and the like may be called "XX information" to indicate that they do not depend on a data structure. When describing identification information, expressions such as "identification information", "identifier", "name", "ID", and "number" are used, but these can be replaced with each other.

When there is a plurality of components having the same or similar functions, the same reference numerals may be given different suffix numerals for description. However, when it is not necessary to distinguish the plurality of components, the suffix numerals are omitted.

In the following description, a processing performed by executing a program may be described, bus since a determined processing is performed in accordance with the program being executed by a processor (for example, CPU, GPU) while using a storage resource (for example, a memory) and/or an interface device (for example, a communication port) as appropriate, a subject of the processing may be the processor. Similarly, the subject of the processing performed by executing the program may be a controller having a processor, a device, a system, a computer, or a node. The subject of the processing performed by executing the program may also be a calculation unit, and may include a dedicated circuit (for example, an FPGA or an ASIC) for performing a specific processing.

The program may be installed on a device such as a computer from a program source. The program source may be, for example, a program distribution server or a computer-readable storage medium. When the program source is the program distribution server, the program distribution server includes a processor and a storage resource for storing a program to be distributed, and a processor of the program distribution server may distribute the program to be distributed to another computer. In the following description, two or more programs may be implemented as one program, or one program may be implemented as two or more programs.

### [First Embodiment]

### <Biological Reaction Information Processing Device>

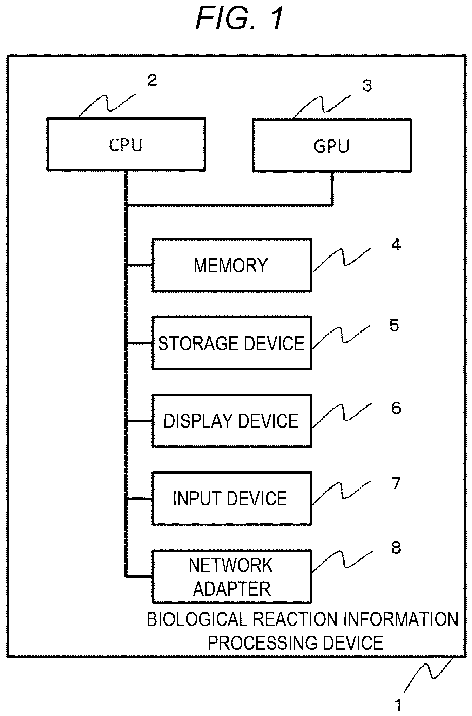

A biological reaction prediction system and a learning method according to the first embodiment will be described with reference to FIGS. 1 to 5.

A biological reaction information processing system according to the present embodiment is mounted on a biological reaction information processing device 1, as shown in FIG. 1. The biological reaction information processing device 1 includes a central processing unit (CPU) 2, a graphic processing unit (GPU) 3, a memory 4, a storage device 5, a display device 6, an input device 7, and a network adapter 8. The CPU 2 and the GPU 3 operate the biological reaction prediction system by executing a program on the memory 4 and processing data stored in the memory 4. The memory 4 includes both a volatile memory and a non-volatile memory. The storage device 5 includes both a hard disk and a solid state drive (SSD). The storage device 5 stores a biological reaction database and a compound database, and is called by the memory 4 as needed. The display device 6 displays data processed by the CPU 2 and data stored in the memory 4 and the storage device 5. The input device 7 is, for example, a mouse, or a keyboard. The network adapter 8 connects the biological reaction information processing device 1 to an external network. The external network is, for example, an Internet or a local area network.

### <Configuration of Biological Reaction Information Processing System>

The biological reaction information processing device 1 according to the present embodiment has a biological reaction information processing system that processes a numerical calculation of a new biological reaction based on a known biological reaction. The biological reaction includes a pre-reaction compound called a substrate, and a post-reaction substance called a product. A synthetic pathway design is an operation of obtaining a pair of this substrate and product by one or more reactions. Biological reactions in the synthetic pathway design include both known and new biological reactions. The biological reaction information processing system according to the present embodiment is for processing information on biological reactions in the synthetic pathway design.

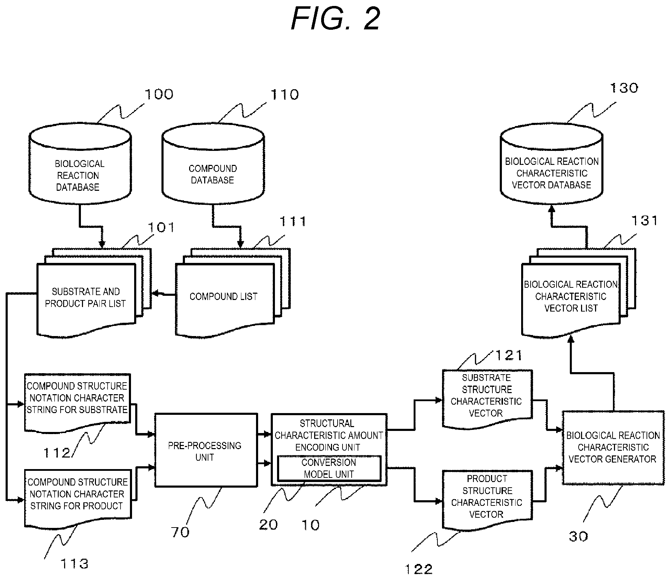

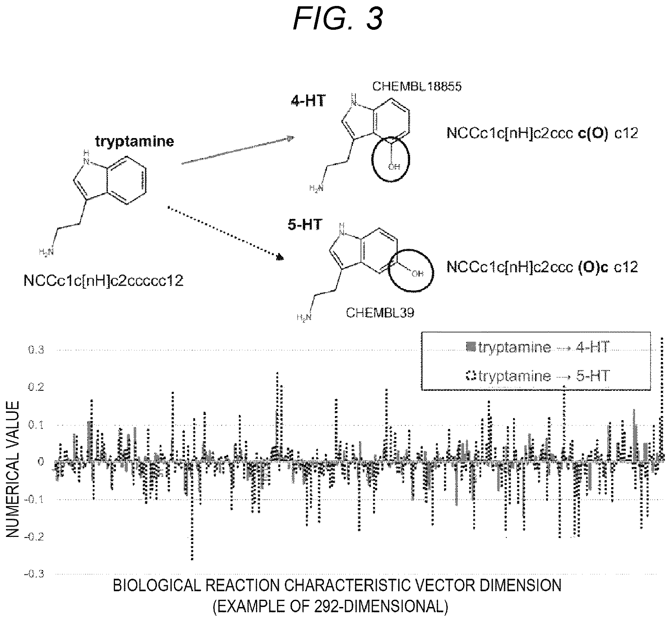

As shown in FIG. 2, the biological reaction information processing system according to the present embodiment includes a biological reaction database 100 in which information on a pair of substrate and product in a biological reaction such as an enzymatic reaction is stored, a compound database 110 in which information on a compound structure is stored, a structural characteristic amount encoding unit 10 that includes a conversion model unit 20 configured to convert a compound structure notation character string of a substrate and a product into a dispersedly represented compound structure characteristic vector (structure characteristic vectors 121 and 122), a biological reaction characteristic vector generator 30 that generates a biological reaction characteristic vector list 131 from the substrate and product structure characteristic vectors, and a biological reaction characteristic vector database 130 that stores the biological reaction characteristic vector list 131. The dispersedly represented compound structure characteristic vector is a fixed-dimensional vector having a plurality of real number values as elements, in which a difference in a structure of one compound is represented by a difference in numerical values of a plurality of real number value elements, and a difference in one real number value element is represented by a difference in a structural change of a plurality of compounds. Similarly, the biological reaction characteristic vector list 131 generated from the substrate and product structure characteristic vectors is also a dispersedly represented characteristic vector. For example, if a biological reaction characteristic vector is represented by 292 (dimensional) real number value elements, the result is as shown in FIG. 3.

The biological reaction database 100 includes, for example, a known metabolic synthesis pathway database such as kyoto encyclopedia of genes and genomes (KEGG) and MetaCyc, and a database created from newly added new metabolic synthesis pathway data and the like. The compound database 110 includes a known compound database such as PubChe, ChEBI, and ZINC, and a database created from newly added new data.

A substrate and product pair list 101 of a biological reaction is created based on the metabolic synthesis pathway data acquired from the biological reaction database 100, and based on information of the compound list 111 acquired from the compound database 110, which is compound information of a biological reaction pair list of the substrate and the product. The compound information includes, for example, a compound structure notation character string such as simplified molecular input line entry system (SMILES), FingerPrint, and MOL format.

### <Function of Biological Reaction Information Processing System: Flow of Generating Biological Reaction Characteristic Vector>

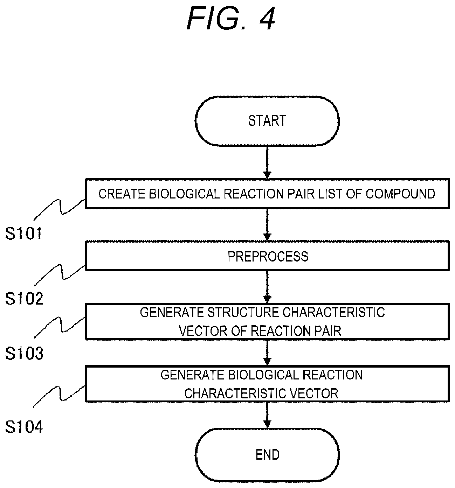

The biological reaction information processing system according to the present embodiment has a function of converting this compound structure into a dispersedly represented numerical vector. With this conversion function, a compound represented by a given number of characters can be uniformly handled as a dispersedly represented numerical vector having at least two or more real number values as elements. If a compound can be uniformly handled as a dispersedly represented numerical vector, a biological reaction can also be represented by a numerical calculation of the numerical vector. Hereinafter, a flow of generating and predicting the biological reaction characteristic vector according to the present embodiment will be described with reference to FIG. 4.

First, a pre-processing unit 70 acquires the substrate and product pair list 101 from the biological reaction database 100 [S101].

Next, the pre-processing unit 70 acquires structure notation character strings 112 and 113 such as SMILES for the substrate and product pair from the substrate and product pair list 101 based on information of the compound list 111 acquired from the compound database 110. Hereinafter, although a description is given using a character string (structure notation character strings 112 and 113) as an example of the structure notation acquired from SMILES and the like, the character string includes identification information such as numbers, symbols, and signs. In other words, the structure notation character strings 112 and 113 are examples of notation information including the character string and identification information.

The pre-processing unit 70 performs a pre-processing for reading the compound structure notation character strings 112 and 113 to input the compound structure notation character strings 112 and 113 to the structural characteristic amount encoding unit 10. The pre-processing unit 70 unifies a SMILES notation of a compound structure, which differs for each database, using RDKit which is open source chemoinformatics software, for example. Next, the pre-processing unit 70 performs a conversion processing so that the SMILES notation is represented by a 1-hot vector used in a language processing program. The pre-processing unit 70 may further incorporate a compound expression grammar function to add a function of removing notations outside a SMILES grammar and a compound expression that cannot exist as an organic compound [S102].

Next, the conversion model unit 20 of the structural characteristic amount encoding unit 10 reads the compound structure notation character string after the pre-processing, and generates the dispersedly represented structure characteristic vectors 121 and 122 having at least two or more elements. Specifically, the conversion model unit 20 has a conversion parameter, and multiplies the compound structure notation character string after the pre-processing by the conversion parameter to generate the structure characteristic vectors 121 and 122 [S103]. The conversion parameter is, for example, a parameter for expressing the compound structure notation character string as a 1-hot vector and the like which is a format usable for learning, and converting the vector into a structure characteristic vector.

The biological reaction characteristic vector generator 30 reads the substrate and product structure characteristic vectors 121 and 122, and generates the biological reaction characteristic vector list 131 [S104].

### <Biological Reaction that cannot be Coped with Existing Technology>

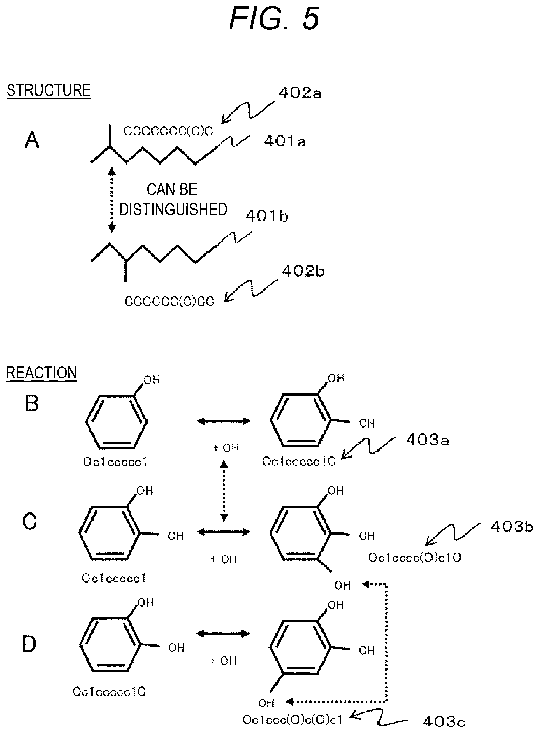

According to the present embodiment, it is possible to handle a compound that cannot be dealt with by an existing technology representing a biological reaction by a calculation such as addition and subtraction of partial structures. FIG. 5 shows characteristics of the biological reaction system according to the present embodiment. For convenience, a virtual reaction will be described as an example.

A method of expressing a compound or a biological reaction as a vector with the number of the partial structures has a problem that the number of elements varies depending on the number of partial structures of the compound, and various reactions cannot be handled uniformly. There is a problem that, in a case where a difference between a substrate structure and a product structure is taken, when structures that remain after performing calculations such as addition and subtraction, that is structures after the biological reactions, that is a difference, are exactly the same structure even for biological reactions that have completely different structures originally, the biological reactions are estimated to be the same.

In the case of a compound as shown in a structure A in FIG. 5, a subject cannot be distinguished because a positional relationship of partial structures is lost.

As shown in reactions B, C, and D in FIG. 5, structures of substrate and product pairs are different, but if the structural difference is taken, the structures are exactly the same. As in C and D in FIG. 5, substrates are the same, but reactions cannot be distinguished when partial structures are connected at a different place after reactions.

On the other hand, in the biological reaction information processing system according to the present embodiment, even when the number of elements is the same, since compounds with different structures are learned as separate compounds by using SMILES and the like written as different character strings, the reactions can be distinguished. For example, in the structure A, structures 401a and 401b are partially defined, and if the defined positional relationship is lost, there is no structural difference before and after the biological reaction, and the two structures cannot be distinguished. However, in the present embodiment, even when the number of elements is the same as in character strings 402a and 402b, compounds having different structures are defined as different character strings, so that both structures can be distinguished. For example, in the reactions B, C, and D, since a difference before and after the reaction is all one "OH", these reactions cannot be distinguished. However, in the present embodiment, even when the number of elements is the same as in character strings 403a, 403b, and 403c, compounds having different structures are defined as different character strings, so that the reactions between them can be distinguished.

As described above, the system includes the structural characteristic amount encoding unit 10 that includes the conversion model unit 20 configured to convert a characteristic amount of notation information indicating chemical structures of a plurality of compounds into a dispersedly represented numerical vector having at least two or more real number values as an element using a conversion parameter, the conversion model unit 20 converting the characteristic amount of the notation information indicating the chemical structures into a numerical vector, for each of a first compound and a second compound among the plurality of compounds, and the biological reaction characteristic vector generator 30 configured to generate a biological reaction characteristic vector between a first compound and a second compound by performing a calculation using a numerical vector of the first compound and a numerical vector of the second compound, thereby performing the above processing.

Therefore, a new biological reaction can be predicted by quantifying while retaining characteristics of an entire compound structure. For example, a variety of compounds, regardless of known or new, can be treated in the same method, and further, the biological reaction can be converted into the characteristic vector while retaining the characteristics of the entire structure, so that an accuracy of the biological reaction prediction in the synthetic pathway design is improved.

### [Second Embodiment]

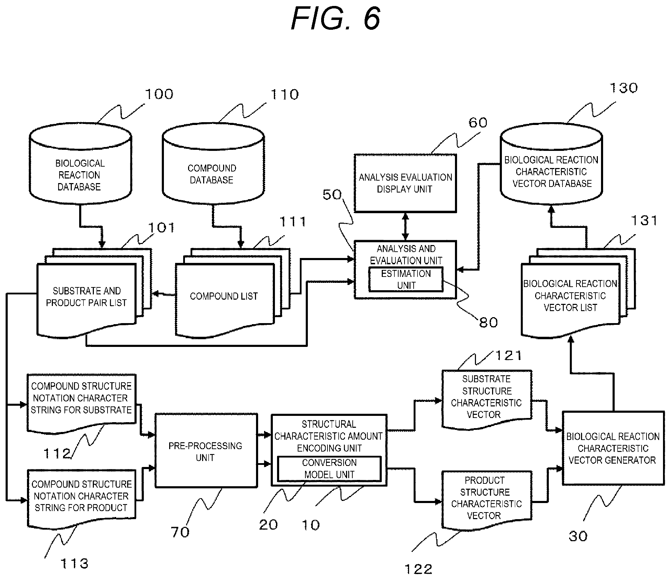

A biological reaction information processing system according to a second embodiment will be described with reference to FIGS. 6 to 11. In the second embodiment, an enzymatic reaction estimation using the biological reaction characteristic vector generated by the biological reaction characteristic vector generator 30 will be described.

### <Function of Biological Reaction Information Processing System: Biological Reaction Prediction Flow>

For a new biological reaction, it is important to calculate a relevance to a known enzymatic reaction. The known enzymatic reaction is labeled with an enzyme number for each reaction. Specifically, International Union of Biochemistry has assigned a four-digit number Z.Z.Z.Z (four sets of numbers separated by dots) beginning with EC.

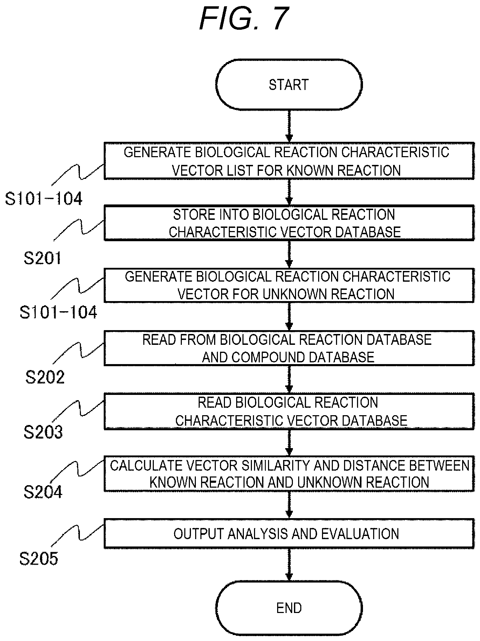

In a biological reaction prediction system according to the present embodiment, an analysis and evaluation unit 50 including an estimation unit 80 is provided as shown in FIG. 6. The analysis and evaluation unit 50 is a processing unit configured to calculate a similarity of biological reactions based on a biological reaction characteristic vector of a known biological reaction. The estimation unit 80 can predict a new biological reaction based on a known enzymatic reaction, for example, by calculating a similarity or a distance between the biological reaction characteristic vectors of the known reaction and a new biological reaction. As will be described later, the estimation unit 80 is configured to, based on the biological reaction characteristic vector of the known biological reaction, perform machine learning on the biological reaction characteristic vector by associating a biological reaction characteristic vector of the same enzymatic reaction group with an enzyme number, and estimate an enzyme number. A calculation of the similarity includes, for example, a cosine similarity, a Jaccard coefficient, a Dice coefficient, and the like, but is not limited to these methods. The calculation of the similarity and the distance makes it possible to estimate a new vector that is close to a known vector in a dispersedly representation space. The biological reaction information processing system described above operates according to a biological reaction prediction flow shown in FIG. 7.

First, the biological reaction characteristic vector list 131 for the known reaction is generated using the structural characteristic amount encoding unit 10 and the biological reaction characteristic vector generator 30 [S101 to S104].

The biological reaction characteristic vector generator 30 stores the biological reaction characteristic vector list 131 related to the known reaction into the biological reaction characteristic vector database 130 [S201].

Next, a biological reaction characteristic vector for a new reaction is generated using the structural characteristic amount encoding unit 10 and the biological reaction characteristic vector generator 30 [S101 to S104].

The analysis and evaluation unit 50 reads a substrate and product pair list and a compound list from the biological reaction database 100 and the compound database 110 [S202].

The analysis and evaluation unit 50 reads the stored known biological reaction characteristic vector database [S203].

The analysis and evaluation unit 50 calculates a similarity and a distance between the vectors of the new reaction and the known reaction [S204]. For the new reaction, a relevance evaluation with the known reaction is analyzed and evaluated, and is output to an analysis evaluation display unit 60 [S205]. For example, the analysis and evaluation unit 50 outputs a result of comparing a similarity between a characteristic vector of a new reaction and a characteristic vector of a known reaction, a result of comparing a similarity between structure vectors, as a result of comparing a similarity between Pathways (combination of vectors), a compound structure characteristic amount, visualization of the biological reaction characteristic vector, and the like to the analysis evaluation display unit 60.

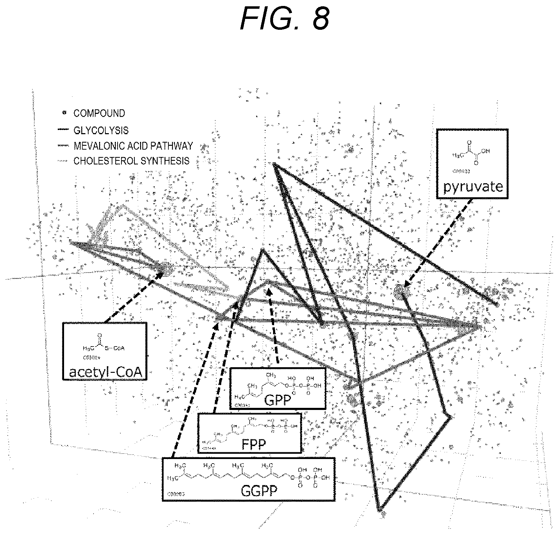

For the visualization of the compound structure characteristic vector and the biological reaction characteristic vector, for example, a method shown in FIG. 8 may be used. FIG. 8 is a diagram in which a compound (correctly, a compound structure characteristic vector) is plotted as a point in a three-dimensional space. A line connecting points is a line connecting a substrate and a product of a certain reaction, and becomes a biological reaction characteristic vector. FIG. 8 is an example in which glycolysis, mevalonic acid pathway, and cholesterol synthesis, which are typical reaction pathways, are displayed.

Compounds having close compound structure characteristic amounts are plotted at a short distance, and compounds having far compound structure characteristic amounts are plotted at a long distance. A plot of the compound structure characteristic vector may be emphasized by, for example, increasing a size of a point according to a frequency of appearance of the compound in the biological reaction database 100. For example, acetyl-CoA and pyruvate in FIG. 8 are frequently appeared compounds, and sizes of plot points are large.

According to the display method as described above, a distance between compounds and a reaction pathway can be intuitively viewed, and visualization that cannot be represented by a reaction map visualized by a related database becomes possible. When the compound structure characteristic vector is two-dimensional or three-dimensional, the plot may be made as it is, but when the vector is four-dimensional or more, a dimensional compression method may be applied. The dimensional compression method may apply, for example, a method such as principal component analysis or t-SNE which is a typical dimensional compression method, but is not limited to these methods.

### <Function of Biological Reaction Information Processing System: Biological Reaction Prediction Using Learning>

In the new reaction, it may be useful to specify the enzyme number up to a third digit. In a first place, a fourth digit is not registered for some known enzymatic reactions.

In the biological reaction information processing system according to the present embodiment, a function of machine learning a biological reaction based on a known biological reaction may be provided, and a function of predicting an enzyme number up to a second or third digit may be provided. For example, based on the known reaction of the biological reaction database 100, the biological reaction characteristic vector database 130 of the known reaction may be learned in association with the enzyme numbers up to the third digit, and may output similar enzyme numbers for biological reactions including the new compound.

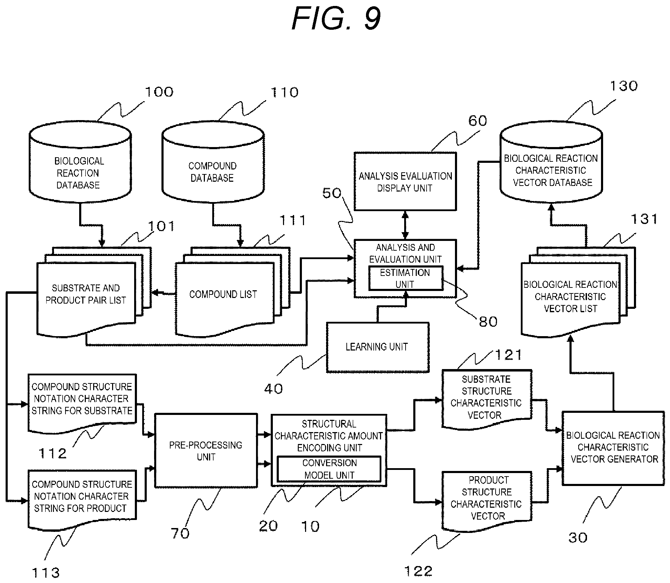

For example, in a form shown in FIG. 9, a learning unit 40 is newly provided, and the estimation unit 80 stores parameters similar to the conversion parameters in the learning unit 40. The learning unit 40 may perform supervised learning for all known biological reactions in association with the enzyme numbers of the substrate and product pair list of the biological reaction characteristic vector database 130 and the biological reaction database 100. The learning unit 40 may use a method such as support vector machine (SVM) or a neural network as a machine learning method for updating the parameters of the estimation unit 80, but is not limited to these methods. As described above, the estimation unit 80 of the analysis and evaluation unit 50 virtually labels at least two or more enzymatic reactions as one enzymatic reaction class, and performs the machine learning.

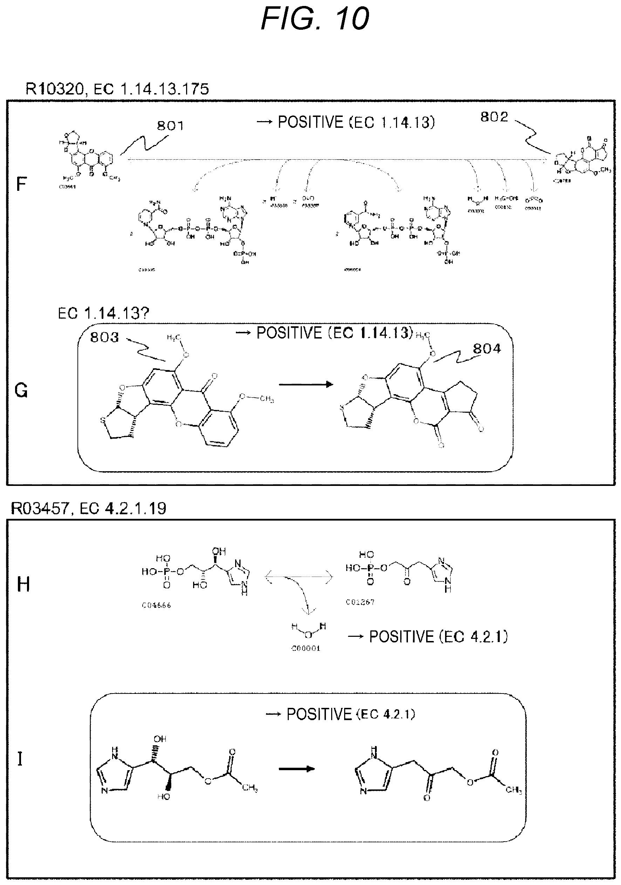

The learning unit 40 performs machine learning using notation information indicating the chemical structures of the plurality of compounds and the biological reaction characteristic vector of the enzymatic reaction group calculated by the estimation unit 80, and updates the conversion parameter of the structural characteristic amount encoding unit 10. An example of an estimation result of an enzymatic reaction in a new biological reaction using the biological reaction information processing system according to the present embodiment will be described with reference to FIG. 10.

A reaction F in FIG. 10 is a reaction in which NADH or NADPH of an enzyme number EC 1.14.13 is used as one electron donor and one oxygen atom is incorporated. In addition to the known EC 1.14.13, the reaction matches a recognition result of a virtually defined reaction G as a new reaction. FIG. 10 shows that the enzyme number obtained by inputting a substrate 803 and a product 804 in the new reaction G matches the enzyme number EC 1.14.13 obtained by inputting a substrate 801 and a product 802 in the known reaction F. Similarly, a reaction H is a dehydratase having an enzyme number EC 4.2.1, and is not only a known EC 1.14.13.175 but also matches a recognition result of a virtually defined reaction I as a new reaction. From the above description, the effectiveness of this method is confirmed.

A fourth digit classification is a part related to substrate specificity. Therefore, when it is necessary to estimate the enzyme number of the new compound up to a fourth digit, after estimating the enzyme number up to the third digit, it is preferable to register a known reaction at the fourth digit in association with a label at the third digit of the enzyme number. For example, by obtaining a structural similarity between a known substrate or product and a substrate or product of a new enzymatic reaction and estimating the fourth digit, an enzyme number close to the new enzymatic reaction can be obtained.

### <New Definition of Known Biological Reaction Used for Learning>

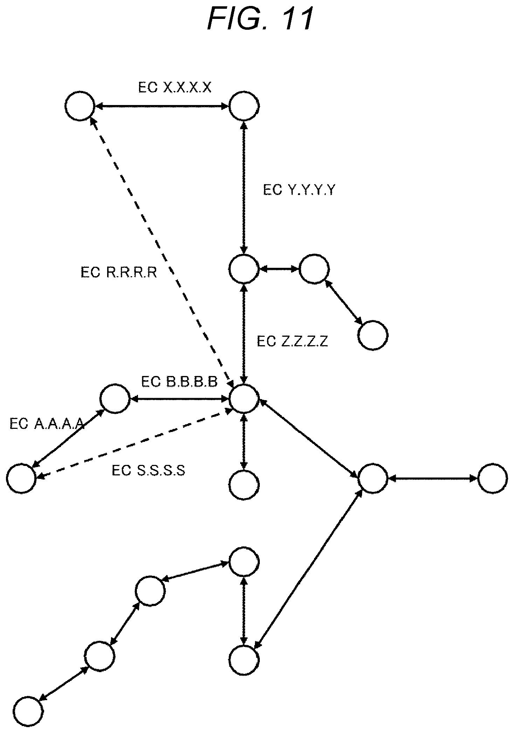

As shown in FIG. 11, in a known synthetic pathway of the biological reaction database 100, one node corresponds to a substrate or a product, and a known reaction or a new reaction corresponds to an edge (arrow). In FIG. 11, a solid line represents a known reaction, and a dotted line represents a new (virtual) reaction.

In the biological reaction prediction system according to the present embodiment, as shown in FIG. 11, the learning unit 40 may define an enzymatic reaction in which a plurality of enzymatic reactions are collected for a certain synthetic pathway and use the enzymatic reaction for learning. For example, FIG. 11 shows an example in which enzymatic reactions of EC X.X.X.X, EC YYYY and EC Z.Z.Z.Z are defined as a new reaction of EC R.R.R.R, and enzymatic reactions of EC A.A.A.A and EC B.B.B.B are defined as a new reaction of EC S.S.S.S.S. EC R.R.R.R and EC S.S.S.S are given fictitious numbers. The learning unit 40 collectively redefines a plurality of enzymatic reactions, thereby making it possible to perform a pathway design while retaining a specific pathway (continuous enzymatic reactions).

### [Third Embodiment]

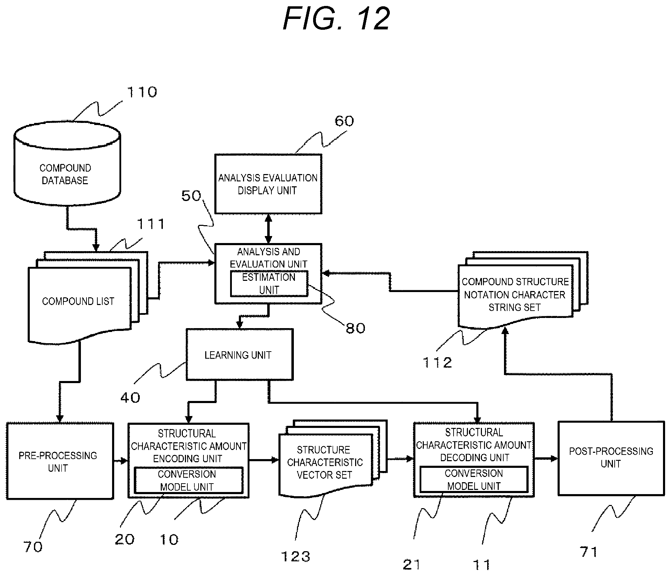

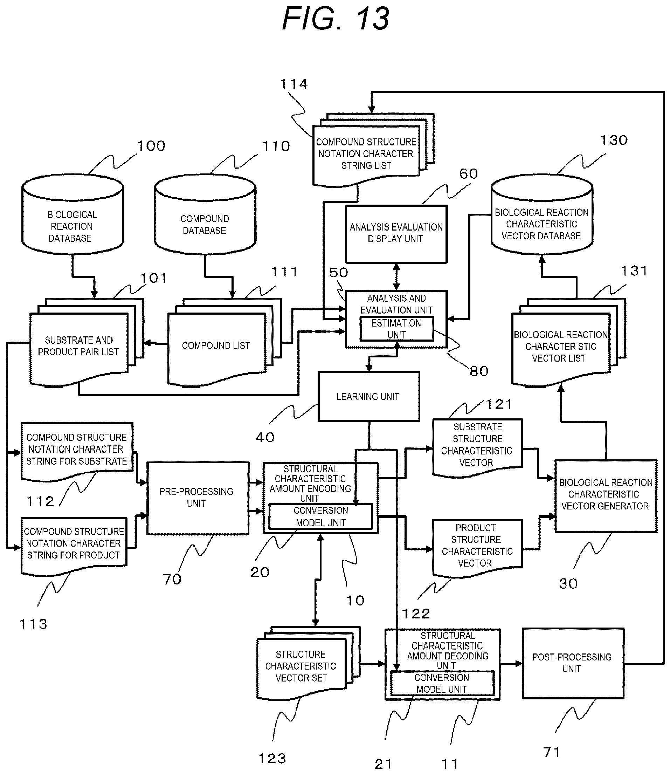

A biological reaction information processing system according to a third embodiment will be described with reference to FIGS. 12 and 13.

### <Learning of Structural Characteristic Amount Encoding Unit: Pre-learning>

The biological reaction information processing system according to the present embodiment generates a compound structure characteristic vector that captures a continuous structural change from a compound structure character string based on an input to the structural characteristic amount encoding unit 10. In the biological reaction information processing system that generates the compound structure characteristic vector, the learning unit 40 may obtain parameters of the conversion model unit 20 in the structural characteristic amount encoding unit 10 by machine learning in advance. For example, it is preferable to use a machine learning technique such as the SVM or the neural network.

A continuous generation of the compound structure characteristic amount may use a method such as variational auto encoder (VAE) or generative adversarial network (GAN), which is a technology derived from the neural network, but is not limited to these methods. For example, when VAE (Gomez-Bombarelli, Rafael, et al. "Automatic chemical design using a data-driven continuous representation of molecules." ACS central science 4.2 (2018): 268-276; Non-Patent Literature 1) is used, learning can be performed as shown in FIG. 12. A structural characteristic amount decoding unit 11 that includes a conversion model unit 21 and a post-processing unit 71 are newly provided.

The pre-processing unit 70 determines whether notation information indicating a compound structure, which is input to the structural characteristic amount encoding unit 10, is a chemically organically positive compound notation. When the pre-processing unit 70 determines that the notation information is a chemically organically positive compound notation, the conversion model unit 21 of the structural characteristic amount decoding unit 11 inputs the structure characteristic vector generated from the structural characteristic amount encoding unit 10 and converts the vector into compound structure notation information. The post-processing unit 71 determines whether the compound structure notation information output from the structural characteristic amount decoding unit 11 is a chemically organically positive compound notation.

First, the pre-processing unit 70 extracts the compound list 111 in which the compound structure is described from the compound database 110, and converts the compound list into a format that can be input to a computer such as a predetermined compound structure notation and a 1-hot vector expression.

The conversion model unit 20 of the structural characteristic amount encoding unit 10 generates a structure characteristic vector set 123 from vectors output from the pre-processing unit 70. The structure characteristic vector set 123 includes structure characteristic vectors of the substrate and the product.

Next, the conversion model unit 21 of the structural characteristic amount decoding unit 11 reads the structure characteristic vector set 123 and generates a compound structure notation character string set 112 via the post-processing unit 71 that returns the vector to a predetermined compound structure notation. The analysis and evaluation unit 50 reads the compound list 111 input to the pre-processing unit 70 and the compound structure notation character string set 112 output by the post-processing unit 71. The learning unit 40 adjusts the parameters of the conversion model unit of the structural characteristic amount encoding unit 10 so that an input character string and an output character string are the same. With such processing, a matching rate of the structure notation character string can be increased.

### <Learning of Structural Characteristic Amount Encoding Unit: Additional Learning>

Hereinafter, a learning flow of the biological reaction information processing system according to the present embodiment will be described with reference to FIG. 13. FIG. 13 is a configuration diagram for additionally learning the structural characteristic amount encoding unit in the biological reaction information processing system according to the present embodiment.

The conversion model unit 20 of the structural characteristic amount encoding unit 10 can perform numerical vectorization by dispersedly representing the compound structure by the pre-learning shown in the third embodiment, but in order to further increase an estimation accuracy of the biological reaction, the analysis and evaluation unit 50 may analyze and evaluate the biological reaction characteristic vector database 130, and the learning unit 40 may apply learning feedback to the conversion model unit 20 of the structural characteristic amount encoding unit 10. That is, the analysis and evaluation unit 50 may feed back an error between the notation information indicating a compound structure input to the structural characteristic amount encoding unit 10 and the compound structure notation information output from the structural characteristic amount decoding unit 11 to the conversion model unit 20, and adjust the parameters of the conversion model unit 20 by machine learning so that outputs of the notation information indicating a compound structure input to the structural characteristic amount encoding unit 10 and the compound structure notation information output from the structural characteristic amount decoding unit 11 are the same.

For example, it is desirable that biological reaction characteristic vectors of the same enzymatic reaction group having a three-digit or two-digit enzyme number be a similar vector in the biological reaction information processing system. Therefore, in the adjustment of the parameters of the conversion model unit 20 of the structural characteristic amount encoding unit 10, the analysis and evaluation unit 50 evaluates not only a simple character string error but also the similarity of the biological reaction characteristic vector of the same enzymatic reaction group for the compound in the biological reaction database 100, and if the group is the same, a regularization term that outputs high similarity may be provided and learning may be performed.

### [Fourth Embodiment]

A biological reaction information processing system according to a fourth embodiment will be described.

### <Synthetic Pathway Design>

One reaction prediction of the biological reaction information processing system according to the present embodiment is connected, and a synthetic pathway design of a known biological reaction and a new biological reaction is performed. The conditions are settings of a maximum number of pathways, a target compound, and an initial compound.

However, it is not always necessary to register the initial compound, and if there is no registration, a given representative compound is used as the initial compound.

A calculation of the synthetic pathway design is preferably performed using a linear programming method. As shown in FIG. 11, a plurality of known reactions may be redefined as one known reaction and included in the synthetic pathway design, and whether the reaction is set to one or a plurality of values as a count of the maximum number of pathways is set by the user.

## Claims

1. A biological reaction information processing system, comprising:
a structural characteristic amount encoding unit that includes a conversion model unit configured to convert a characteristic amount of notation information indicating chemical structures of a plurality of compounds into a dispersedly represented numerical vector having at least two or more real number values as an element using a conversion parameter, the conversion model unit converting the characteristic amount of the notation information indicating the chemical structures into a numerical vector, for each of a first compound and a second compound among the plurality of compounds; and
a biological reaction characteristic vector generator configured to generate a biological reaction characteristic vector between the first compound and the second compound by performing a calculation using a numerical vector of the first compound and a numerical vector of the second compound.

2. The biological reaction information processing system according to claim 1, wherein
the conversion model unit sets the numerical vector as a compound structure characteristic vector with a fixed-dimensional vector having a plurality of real number values as elements, in which a difference in a structure of one compound is represented by a difference of a plurality of real number values, and a difference in one real number value is represented by a difference in a structural change of a plurality of compounds.

3. The biological reaction information processing system according to claim 1, comprising:
an analysis and evaluation unit configured to calculate a similarity of biological reactions based on a biological reaction characteristic vector of a known biological reaction.

4. The biological reaction information processing system according to claim 3, wherein
the analysis and evaluation unit includes an estimation unit configured to, based on the biological reaction characteristic vector of a known biological reaction, perform machine learning on the biological reaction characteristic vector by associating a biological reaction characteristic vector of the same enzymatic reaction group with an enzyme number, and estimate an enzyme number.

5. The biological reaction information processing system according to claim 4, wherein
the estimation unit of the analysis and evaluation unit virtually labels at least two or more enzymatic reactions as one enzymatic reaction class, and performs machine learning.

6. The biological reaction information processing system according to claim 4, comprising:
a learning unit configured to perform machine learning using notation information indicating chemical structures of the plurality of compounds and a biological reaction characteristic vector of the enzymatic reaction group calculated by the estimation unit, and update a conversion parameter of the structural characteristic amount encoding unit.

7. The biological reaction information processing system according to claim 1, further comprising:
a pre-processing unit configured to determine whether notation information indicating the compound structure, which is input to the structural characteristic amount encoding unit, is a chemically organically positive compound notation;
a structural characteristic amount decoding unit that includes a conversion model unit configured to convert a structure characteristic vector generated from the structural characteristic amount encoding unit into compound structure notation information as an input; and
a post-processing unit configured to determine whether compound structure notation information output from the structural characteristic amount decoding unit is a chemically organically positive compound notation.

8. The biological reaction information processing system according to claim 7, wherein
an analysis and evaluation unit configured to calculate a similarity of biological reactions based on the biological reaction characteristic vector of a known biological reaction feeds back an error between the notation information indicating a compound structure input to the structural characteristic amount encoding unit and compound structure notation information output from the structural characteristic amount decoding unit to the conversion model unit, and performs machine learning so that outputs of the notation information indicating the compound structure input to the structural characteristic amount encoding unit and the compound structure notation information output from the structural characteristic amount decoding unit are the same.

9. A biological reaction information processing method, comprising:
converting a characteristic amount of notation information indicating chemical structures of a plurality of compounds into a dispersedly represented numerical vector having at least two or more real values as an element using a conversion parameter by an conversion model unit of a structural characteristic amount encoding unit;
converting the characteristic amount of the notation information indicating the chemical structures into a numerical vector for each of a first compound and a second compound among the plurality of compounds by the conversion model unit; and
generating a biological reaction characteristic vector between the first compound and the second compound by performing a calculation using a numerical vector of the first compound and a numerical vector of the second compound by a biological reaction characteristic vector generator.
